# EUROPEAN PATENT SPECIFICATION

(11) **EP 2 034 969 B1**
(45) Date of publication and mention of the grant of the patent: **11.01.2017**
(21) Application number: 07796595.2
(22) Date of filing: 29.06.2007
(51) Int. Cl.: A61K 9/20, A61K 31/4418, A61K 31/444, A61K 31/5377

(54) **IMMEDIATE-RELEASE TABLET FORMULATIONS OF A THROMBIN RECEPTOR ANTAGONIST**
TABLETTENFORMULIERUNGEN EINES THROMBIN-REZEPTOR-ANTAGONISTEN MIT SOFORTIGER FREISETZUNG
PRÉPARATIONS EN COMPRIMÉ À LIBÉRATION IMMÉDIATE D'UN ANTAGONISTE DES RÉCEPTEURS DE LA THROMBINE

(30) Priority: 30.06.2006 US 817821 P
(43) Date of publication of application: 18.03.2009
(60) Divisional application: 12159636.5
(73) Proprietor: Merck Sharp & Dohme Corp., Rahway, NJ 07065-0907 (US)
(72) Inventor: GUPTA, Rajan, Bridgewater, NJ 08807 (US); CHAWDRY, Suliman, South Plainfield, NJ 07080 (US); DUGGIRALA, Srinivas, S., Raritan, NJ 08869 (US)
(74) Representative: Buchan, Gavin MacNicol
(86) International application number: PCT/US2007/015168
(87) International publication number: WO 2008/005353

(56) References cited:
- WO-A-2007/117621
- US-A1- 2003 216 437
- US-A1- 2004 176 418

## Description

### FIELD OF THE INVENTION

The invention relates to immediate-release tablet formulations for delivery of loading and maintenance doses of a thrombin receptor antagonist as set forth in the claims.

### BACKGROUND

Thrombin is known to have a variety of activities in different cell types and thrombin receptors are known to be present in such cell types as human platelets, vascular smooth muscle cells, endothelial cells and fibroblasts. It is therefore possible that thrombin receptor antagonists, also known as protease activated receptor (PAR) antagonists will be useful in the treatment of thrombotic, inflammatory, atherosclerotic and fibroproliferative disorders, as well as other disorders in which thrombin and its receptor play a pathological role.

Thrombin receptor antagonists have been suggested in the literature as being potentially useful in treating a variety of cardiovascular diseases or conditions including, for example, thrombosis, vascular restenosis, deep venous thrombosis, lung embolism, cerebral infarction, heart disease, disseminated intravascular coagulation syndrome, hypertension (Suzuki, Shuichi, PCT Int. Appls. WO 0288092 (2002), WO 0285850 (2002) and WO 0285855 (2002)), arrhythmia, inflammation, angina, stroke, atherosclerosis, ischemic conditions (Zhang, Han-cheng, PCT Int. Appl. WO 0100659 (2001), WO 0100657(2001) and WO 0100656 (2001)).

U.S. Application No. 10/412,982 discloses a specific thrombin receptor antagonist compound identified as Example 2, herein identified as COMPOUND 1. COMPOUND 1 has the following structure:

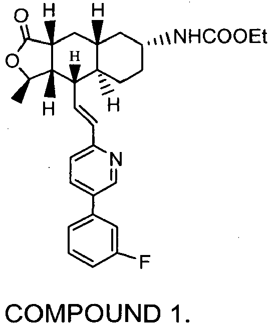

COMPOUND 1 exhibits good thrombin receptor antagonist activity (potency) and selectivity, and the bisulfate salt of COMPOUND 1 is currently in development by Schering Corp. A crystalline form of the bisulfate salt of COMPOUND 1 is disclosed in U.S. pat. no. 7,235,567.

The use of a small subset of thrombin receptor antagonists to treat a variety of conditions and diseases is disclosed in U.S. publication no. 04/0192753. The prevention of complications associated with cardiopulmonary bypass surgery by administration of a thrombin receptor antagonist is taught in U.S. application no. 11/613,450. Substituted thrombin receptor antagonists are disclosed in US patent nos. 6,063,847; 6,326,380; and 6,645,987 and U.S. publication nos. 03/0203927; 04/0216437A1; 04/0152736; and 03/0216437.

It would be beneficial to provide a set of thrombin receptor antagonist immediate-release formulations of acceptable dissolution characterics, including such formulations of COMPOUND 1. The invention seeks to provide these and other benefits, which will become apparent as the description progresses.

### SUMMARY OF THE INVENTION

The present invention is directed to a solid pharmaceutical formulation for oral administration comprising COMPOUND 1 or a pharmaceutically acceptable salt thereof and at least one disintegrant, wherein the amount of COMPOUND 1 is less than 7% of the weight of the formulation, wherein the ratio of disintegrant to COMPOUND 1 or a pharmaceutically acceptable salt or solvate thereof is between 0.6 and 12 on a weight/weight basis, wherein the weight of COMPOUND 1 or a pharmaceutically acceptable salt or solvate thereof is between 0.5 mg and 10 mg and the total weight of the formulation is between 100 mg and 400 mg.

In some embodiments, the formulation is a tablet.

In some embodiments, the ratio of disintegrant to COMPOUND 1 is 0.75 and 1.0. In some embodiments, the ratio is 0.9. In some embodiments, the ratio is 2.4.

In some embodiments, the weight of COMPOUND 1 is about 2.5 mg and the total weight of the formulation is about 100 mg.

In some embodiments, the COMPOUND 1 is a bisulfate salt.

In some embodiments, the formulation results in a 30-minute dissolution of at least 80%. In some embodiments, the formulation results in a 30-minute dissolution of at least 85%.

In some embodiments, the disintegrant is selected from the group consisting of croscarmellose sodium, starch, sodium starch glycolate, crospovidone and microcrystalline cellulose. In some embodiments, the disintegrant is croscarmellose sodium.

In some embodiments, the formulation further comprises at least one diluent, at least one binder and at least one lubricant. In some embodiments, the diluent is selected from one or more of the group consisting of lactose monohydrate, microcrystalline cellulose, mannitol, sorbitol, tribasic calcium phosphate, diabasic calcium phosphate, compressible sugar, starch, and calcium sulfate. In some embodiments, the diluent is selected from one or more of the group consisting of lactose monohydrate and microcrystalline cellulose.

In some embodiments, the binder is selected from the group consisting of povidone, acacia, tragacanth, hydroxypropylcellulose, pregelatinized starch, gelatin, hydroxypropylcellulose, hydroxypropylmethylcellulose, methylcellulose, sugar solutions, such as sucrose and sorbitol, and ethylcellulose. In some embodiments, the binder is povidone.

In some embodiments, the lubricant is selected from the group consisting of magnesium stearate, stearic acid and talc. In some embodiments, the lubricant is magnesium stearate.

In some embodiments, the formulation comprises 2.5 mg of Compound 1 or a pharmaceutically acceptable salt thereof and at least 5 weight percent of a disintegrant. In some embodiments, the total weight of said formulation is 100 mg.

In some embodiments, the formulation comprises:

| Ingredient | Amount (mg) |
|---|---|
| COMPOUND 1 Bisulfate | 2.5 |
| Lactose Monohydrate | 68 |
| Microcrystalline Cellulose | 20 |
| Croscarmellose Sodium | 6 |
| Povidone | 3 |
| Magnesium Stearate | 0.5. |

In some embodiments, the invention is directed to methods of treating acute coronary syndrome or peripheral arterial disease, or of treating a patient in need of secondary prevention by orally administering to a patient in need of such treating the pharmaceutical formulation.

In some embodiments, the invention is directed to an immediate-release tablet formulation of a thrombin receptor antagonist that results in a 30-minute dissolution of at least 80%, wherein said thrombin receptor antagonist is:

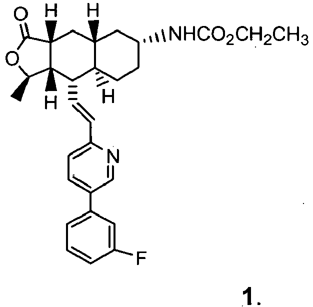

A further understanding of the invention will be had from the following drawings, description and claims.

### BRIEF DESCRIPTION OF THE DRAWINGS

FIG. 1 is a graph of percent dissolution of COMPOUND 1 vs time for tablet formulations of various API loadings.
FIG. 2 is a graph of percent dissolution of COMPOUND 1 after 30 minutes for various prototype tablet formulations.
FIG. 3 is a graph of percent dissolution of COMPOUND 1 vs time of controlled disintegrant-to-API ratio prototype tablet formulations.
FIG. 4 is a graph of percent dissolution of COMPOUND 1 vs time of controlled disintegrant concentration prototype tablet formulations.

### DETAILED DESCRIPTION

Schering Corp. is developing a thrombin receptor antagonist for use in a variety of cardiovascular applications, including acute coronary syndrome and prevention of later coronary events subsequent to initial coronary events ("secondary prevention"). The active pharmaceutical ingredient ("API"), COMPOUND 1, has been evaluated in phase II clinical trials. Dosing regimens being considered for commercialization include potential loading doses of 10, 20 and 40 mg and maintenance doses of 0.5, 1, 2.5 and 5 mg, in solid, immediate-release tablet formulations for oral administration. Immediate-release formulations are sought in order to ensure rapid delivery of a thrombin receptor antagonist to the patient. In the case of a patient who may have just suffered an acute coronary event (*e.g*., a stroke), and who is thus at risk for serious imminent further cardiovascular consequences (*e.g*., coronary ischemia), rapid delivery of a loading dose of the thrombin receptor antagonist may be crucial. It is believed that the risk of such cardiovascular consequences can be mitigated by rapidly delivering to such a patient a therapeutically effective amount of a thrombin receptor antagonist, and that this can be achieved by an immediate-release formulation of acceptable pharmaceutical characteristics. Based on clinical data, it appears that a loading dose of 20 or 40 mg will safely achieve therapeutically effective blood levels of COMPOUND 1 in a patient in the desired time frame. Thus, the development of formulations of suitable pharmaceutical characteristics is a necessary step in the commercialization of this thrombin receptor antagonist.

In preparation for phase II clinical trials (and prior to the appreciation gained from examination of the results of those trials that distinct loading and maintenance doses would be appropriate), several immediate-release formulatons were prepared. Formula selection was based on dissolution results from formulation screening studies to support the formulation content uniformity/assay, active/excipient compatibility studies, and longer term data from stability screening trials. The manufacturing processes for these formulations involve the steps of wet granulation, drying, blending, and compression, followed by an optional film-coating operation. Table 1 displays the formulations of COMPOUND 1 bisulfate tablets of doses of 0.5, 1, 2.5, 10 and 20 mg.

**Table 1.**

| **Ingredient** | **Function** | **Theoretical mg/tablet** | | | | | | |
|---|---|---|---|---|---|---|---|---|
| | | **0.5 mg Tablet^{*}** | **1 mg Tablet^{*}** | **2.5 mg Tablet^{*}** | **10 mg Tablet^{*}** | **20 mg Tablet** | **10 mg Tablet** | **10 mg Tablet*** |
| Formulation No. → | | 1A | 1B | 1C | 1D | 1E | 1F | 1G |
| COMPOUN D1 bisulfate | Active | 0.5 | 1.0 | 2.5 | 10.0 | 20.0 | 10.0 | 10.0 |
| Lactose Monohydrat | Diluent | 70 | 69.5 | 68.0 | 272.0 | 262.0 | 60.5 | 131.0 |
| e (Impalpable Powder) | | | | | | | | |
| Microcrystall ine Cellulose | Diluent | 20.0 | 20.0 | 20.0 | 80.0 | 80.0 | 20.0 | 40.0 |
| Croscarmell ose Sodium | Disintegrant | 6.0 | 6.0 | 6.0 | 24.0 | 24.0 | 6.0 | 12.0 |
| Povidone K-30 | Binder | 3.0 | 3.0 | 3.0 | 12.0 | 12.0 | 3.0 | 6.0 |
| Magnesium Stearate | Lubricant | 0.5 | 0.5 | 0.5 | 2.0 | 2.0 | 0.5 | 1.0 |
| Purified Water | Solvent | (---)^{a} | (---)^{a} | (---)^{a} | (---)^{a} | (---)^{a} | (---)^{a} | (---)^{a} |
| **Theoretical Total Core Tablet Weight** | | **100.0** | **100.0** | **100.0** | **400.0** | **400.0** | **100.0** | **200.0** |
| Opadry Coating System | Coating Agent | See footnote b. | | | | | | |

| | | | | | | | | |
|---|---|---|---|---|---|---|---|---|
| a: Evaporates during the drying and coating processes. b: Coating is optional, but may be used for cosmetic reasons. Expected coating level is between 2.5 - 10%, preferably between 3 - 6%. Applies to all formulations herein. *: Inventive formulation. | | | | | | | | |

These prototype tablets were tested for standard pharmaceutical quality attributes, including dissolution (employing a basket type dissolution apparatus equipped with a suitable ultraviolet spectrophotometer, with agitation at 50 rpm and an acidic dissolution test media containing 0.05N HCI).

In phase III clinical trials planned for the evaluation of COMPOUND 1 in the treatment of acute coronary syndrome and secondary prevention, the 2.5 mg dose of Formulation 1C is planned for administration as a maintenance dose.

"Acute coronary syndrome" includes any group of clinical symptoms compatible with acute myocardial ischemia. Acute myocardial ischemia is chest pain due to insufficient blood supply to the heart muscle that results from coronary artery disease (also called coronary heart disease). Acute coronary syndrome thus covers the spectrum of clinical conditions ranging from unstable angina to non-Q-wave myocardial infarction and Q-wave myocardial infarction. Symptoms may include chest pain, shortness of breath, nausea, vomiting, diaphoresis (sweating), palpitations, anxiety or a sense of impending doom and a feeling of being acutely ill.

"Secondary prevention" refers to the treatment of patients who have already suffered a significant cardiovascular event, such as a heart attack or stroke, to prevent another future, potentially more serious, perhaps lethal, cardiovascular or cerebrovascular event.

Another cardiovascular condition for which thrombin receptor antagonists may be useful is peripheral arterial disease ("PAD"), also known as peripheral vascular disease ("PVD"), which occurs when cholesterol and scar tissue build up, forming plaque inside the arteries that narrows and clogs the arteries. The clogged arteries cause decreased blood flow to the legs, which can result in pain when walking, and eventually gangrene and amputation.

One pharmaceutical characteristic that is always important in orally administered formulations is rate of dissolution. Typical immediate-release formulation specifications require that not less than 75-80% of the active be dissolved within a 30-minute period. In certain formulations similar to those enumerated in Table 1, an undesired reduction in dissolution rates was detected regarding some formulated tablets after having been subjected to stability evaluation. In particular, a significant change in dissolution rate profiles was noted between initial and 1-month stability 10 mg batches (formulated to a total formulation weight of 100 mg).

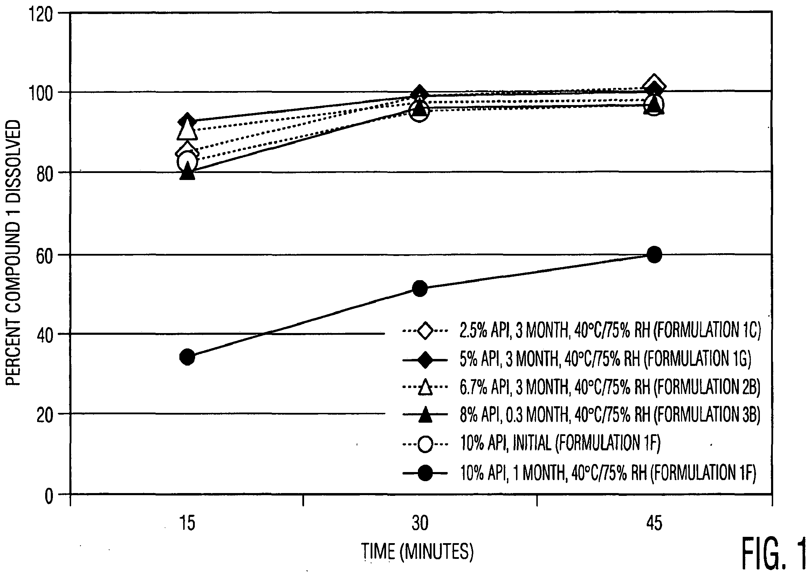

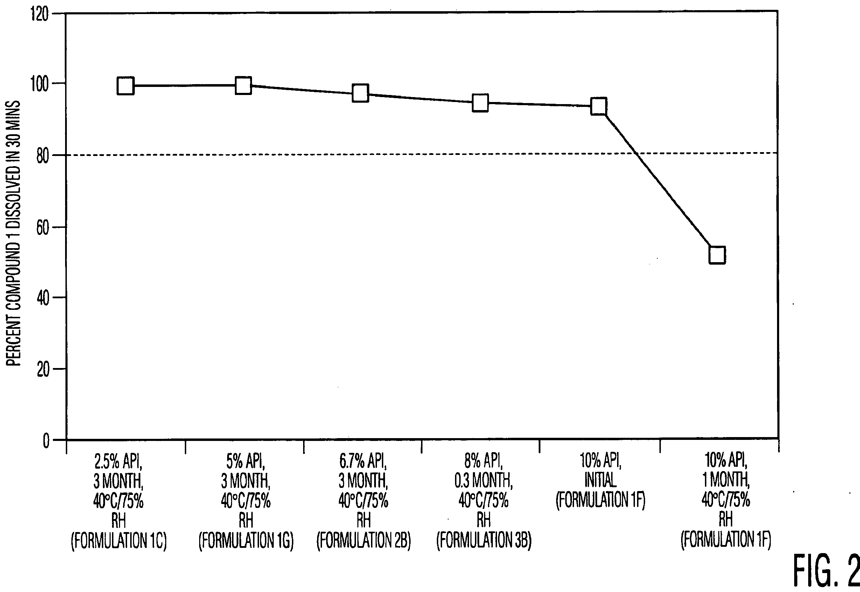

One parameter which can affect dissolution of the active in a solid dosage form is API loading (*i.e*., the weight ratio of API to the total tablet core). To determine whether API loading was a factor in the drop in dissolution displayed in some of the COMPOUND 1 tablets, several formulations of varying API loading were prepared and dissolutions were then measured for both fresh batches and those having been in various stability conditions. The results are displayed in FIGS. 1 and 2. The dissolution data suggest that at high temperature/humidity conditions API loading should be less than 10% in order to meet the 80% dissolution criterion. An API loading of as high as 8% was found to meet this criterion, and the data suggest that it would be possible to exceed 8% without failing the 80% dissolution criterion, if required. It was concluded that with futher exploration of the excipients and their levels, an API loading in excess of 10%, perhaps up to about 12%, could be engineered into a tablet that would demonstrate satisfactory dissolution characteristics.

After pharmacokinetic data from phase II clinical trials were examined, it was determined that a loading dose of COMPOUND 1 may be appropriate, and that this loading dose may be in the range of 20 to 40 mg. A loading dose of 40 mg is planned for evaluation in phase III clinical trials. The question facing the formulators was what size tablet would be required, particularly for the 40 mg formulation. For higher dose tablets, higher API loadings may be desirable in order to achieve reasonable tablet size, avoid content uniformity issues, and control the cost of goods sold. Thus for the 40 mg formulation, additional understanding of the dissolution characteristics of COMPOUND 1 was sought.

The dissolution data also suggest that API loading was not the sole factor in the observed dissolution slow-down. It was hypothesized that the dissolution slow down was related to the ratio of disintegrant to API and moisture. Hence dissolution could be controlled by adjusting the ratio of disintegrant to API in the formulation.

For initial evaluation of the effects of disintegrant-to-API ratio and moisture content, three preliminary formulation prototype tablets were initially developed. Each tablet contained 40 mg of COMPOUND 1 bisulfate, and the tablets weighed a total of 400, 600, and 800 mg, respectively. Each of the prototypes contained identical percentages of the following inactive excipients: Microcrystalline Cellulose as a diluent (20%), Croscarmellose Sodium as a disintegrant (6%), Povidone as a binder (3%), and Magnesium Stearate as a lubricant (0.5%). The amount of Lactose Monohydrate as a diluent was varied in each formulation based upon the total tablet weight and the sum total of the individual excipient amounts listed above. Table 2 displays these three prototype formulations.

**Table 2.**

| **Ingredient** | **Function** | **Concentration (mg/tablet)** | | |
|---|---|---|---|---|
| **Formulation No. →** | | **2A** | **2B** | **2C** |
| COMPOUND 1 Bisulfate | API | 40 | 40 | 40 |
| Lactose Monohydrate | Diluent | 242 | 383 | 524 |
| Microcrystalline Cellulose | Diluent | 80 | 120 | 160 |
| Croscarmellose Sodium | Disintegrant | 24 | 36 | 48 |
| Povidone | Binder | 12 | 18 | 24 |
| Magnesium Stearate | Lubricant | 2 | 3 | 4 |
| **Total** | | **400** | **600** | **800** |
| **Disintegrant/API** | | **0.6:1** | **0.9:1** | **1.2:1** |
| **API Loading (API/Total)** | | **0.1** | **.067** | **.05** |

These prototype tablets were tested for standard pharmaceutical quality attributes, including dissolution (employing a Distek 2100/5100 paddle type dissolution apparatus equipped with a suitable ultraviolet spectrophotometer, with agitation at 50 rpm and an acidic dissolution test media containing 0.01 N HCl).

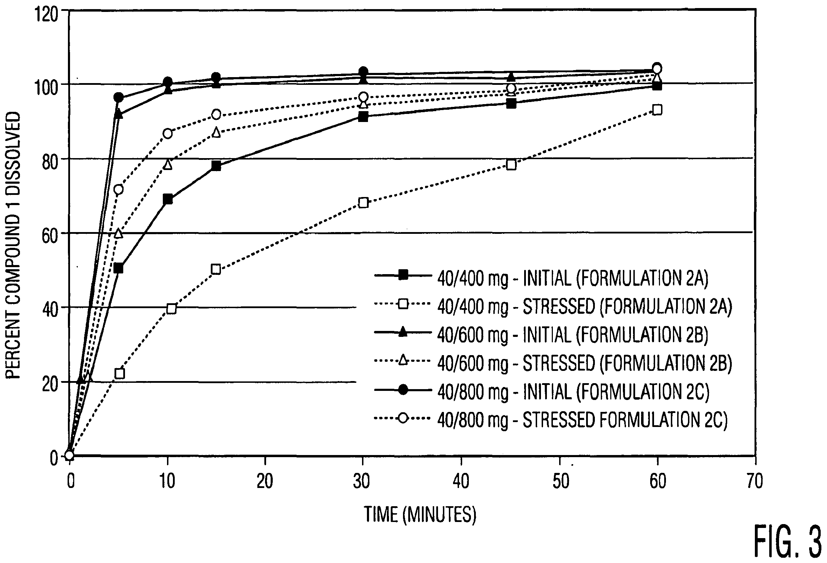

Additionally, tablet samples were also stored under stressed conditions (*i.e*., 40°C temperature and 75% relative humidity) and tested for dissolution. The results of dissolution rate analyses of both standard and stressed samples of each of the three initial prototype formulations are displayed in FIG. 3. It is of note that some of the dissolution data associated with later time points display values greater than the theoretical label claim of 100%. This is attributed to variability in both the manufacturing process and the dissolution testing methodology. As a reference, a majority of immediate-release pharmaceutical aritcles have commercial assay specification ranges of 95-105% of the stated label claim.

Inspection of the dissolution data leads to the following observations. The 400 mg tablets (**1A**) exhibited a significant drop in dissolution after being exposed to accelerated temperature and humidity conditions, *i.e*., upon uptake of increased amounts of moisture. Approximately 68% of the API had dissolved within 30 minutes from the stressed 400 mg formulation, as compared to >95% from all the other samples. The stressed 400 mg formulation is the only one that failed to meet the 30-minute standard of 75-80% dissolution. Visual observations of the stressed 40/400 mg test samples revealed inadequate disintegration of the tablet granules into primary API and excipient particles. In addition, a gel-like layer was found to exist on the surface of the tablet granules, theorized to be related to moisture uptake. Based upon these observations, the hypothesis of the combination of the disintegrant-to-API ratio and moisture content of the tablets affecting dissolution rates in these COMPOUND 1 formulations was further supported.

In order to test this hypothesis, a series of experiments involving both positive and negative controls was devised. Three additional prototype tablets were formulated as follows:
∘ 400 mg tablets containing disintegrant at a 10% level, with a disintegrant-to-API ratio of 1:1. This prototype served as a positive control, *i.e*., to assess whether the dissolution rate of a previously "failing" sample could be increased by increasing the disintegrant-to-API ratio, all other factors being equal.
∘ 500 mg tablets containing disintegrant at a nominal 6% level, with a disintegrant-to-API ratio of 0.75.
∘ 800 mg tablets containing disintegrant at a 3% level, to yield a disintegrant-to-API ratio of 0.6. This prototype served as a negative control, *i.e*., to assess whether the dissolution rate of a previously "passing" sample could be decreased by decreasing the disintegrant-to-API ratio.

Table 3 displays these three additional prototype formulations as 3A, 3B and 3C.

**Table 3.**

| **Ingredient** | **Function** | **Concentration (mg/tablet)** | | |
|---|---|---|---|---|
| **Formulation No. →** | | **3A** | **3B** | **3C** |
| COMPOUND 1 Bisulfate | API | 40 | 40 | 40 |
| Lactose Monohydrate | Diluent | 234 | 588 | 588 |
| Microcrystalline Cellulose | Diluent | 72 | 160 | 160 |
| Croscarmellose Sodium | Disintegrant | 40 | 24 | 24 |
| Povidone | Binder | 12 | 24 | 24 |
| Magnesium Stearate | Lubricant | 2 | 4 | 4 |
| Water | Solvent | -^{a} | -^{a} | -^{a} |
| **Total** | | **400** | **800** | **800** |
| **Disintegrant/APl** | | **1:1** | **0.75:1** | **0.6:1** |
| **API Loading (API/Total)** | | **.10** | **.08** | **0.05** |

| | | | | |
|---|---|---|---|---|
| a: Evaporates during the manufacturing process | | | | |

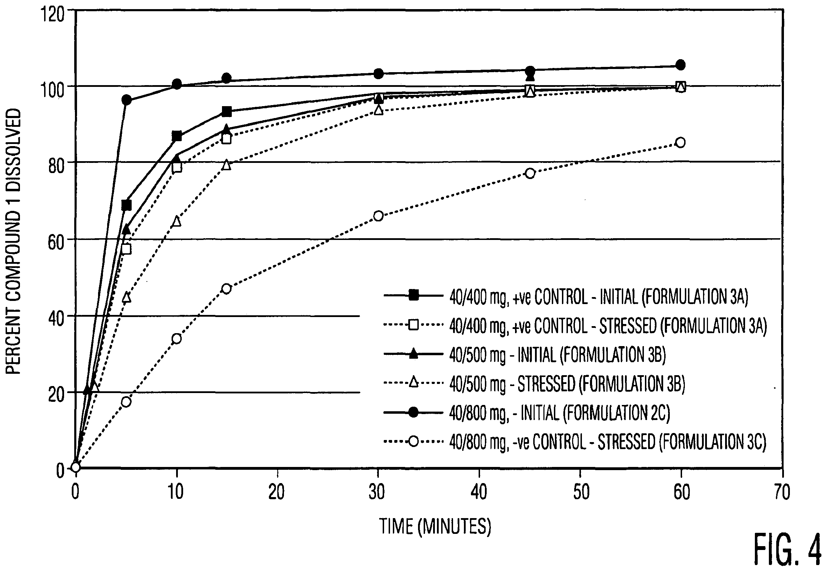

These additional prototypes were also stored under standard and accelerated conditions, as referenced above. Dissolution profiles of all three prototypes are displayed in FIG. 4. The data show that the dissolution rates of the stressed 40/400 mg tablets were significantly improved by increasing the disintegrant-to-API ratio to 1:1 (comparing FIGS. 3 and 4). Conversely, by decreasing the disintegrant-to-API ratio of the stressed 40/800 mg tablets, the dissolution rate was significantly retarded. These data suggest that manipulation of the moisture content of the tablets and the disintegrant-to-API ratio within the formulation has an influence on tablet dissolution rates.

Based upon the results of the studies described above, it was concluded that, for the 40 mg dose formulation, a threshold disintegrant-to-API ratio was warranted in order to minimize moisture-mediated drop in dissolution under standard pharmaceutical product storage conditions. As shown in FIG. 4, the 400 and 500 mg formulations (**3A** and **3B,** respectively) have sufficiently robust dissolution profiles, displaying greater than 95% and 90% dissolutions after 30 minutes, respectively. Based on the above, and a desire to achieve superior dissolution characteristics in a reasonably sized tablet, a 600 mg tablet weight (Formulation **2B** in Table 2) was selected as the 40 mg dose formulation for administration in phase III clinical trials planned for the evaluation of COMPOUND 1 in the treatment of acute coronary syndrome and secondary prevention.

A range of formulations is within the scope of this invention. The maintenance dose of COMPOUND 1 can be varied within a range of 0.5 to 10 mg, preferably 1 to 2.5 mg. The amounts of individual excipients in the formulation can be adjusted within acceptable ranges, as understood by those skilled in the art.

These tablets are manufactured via a process involving high-shear wet granulation with an aqueous povidone solution, drying the granulation to a final moisture content of 0.5-2.0% in a fluid-bed processor, blending with the referenced lubricant in a tumble blender or equivalent, and compressing on a rotary tablet press into tablets of the desired weight.

By way of example, a manufacturing process for the 1 mg formulation (**1B**) is as follows:
1. Dissolve the povidone in purified water. Mix until a clear solution is obtained.
2. Pass the COMPOUND 1 bisulfate, lactose monohydrate, microcrystalline cellulose, and croscarmelose sodium through suitably sized screen(s)
3. Charge the screened ingredients from Step 2 into a suitably sized granulator and blend.
4. Spray the povidone solution from Step 1 on to the blend. Additional water may be added to achieve a satisfactory granulation. Mix the wet grantulation in the granulator.
5. Pass the granulation through a screen into a suitably sized fluid bed processor.
6. Dry the granulation until a suitable loss on drying is achieved.
7. Pass the dried granulation through a screen into a suitably sized tumble blender.
8. Pass the magnesium stearate through a screen into a suitably sized screen into the blender from Step 7 and blend.
9. Compress the tablets from Step 9 into a suitable sized pan coater.
10. Prepare a suspension of Opadry II in purified water.
11. Coat the tablets using the suspension from Step 11.

The specific diluents, disintegrants, binders and lubricants listed above are not thought to be exclusively applicable to providing acceptable pharmaceutical characteristics in formulations of COMPOUND 1, and other functional equivalents may be substituted for those listed. Preferred diluents comprise lactose, including lactose monohydrate (impalpable powder), microcrystalline cellulose (*e.g*., Avicel PH 102), mannitol, sorbitol, tribasic calcium phosphate, diabasic calcium phosphate, compressible sugar, starch, and calcium sulfate. Lactose monohydrate originates from bovine sources and can be obtained from Foremost Farms. Preferred binders comprise povidone (*e.g*., PVP K-30), acacia, tragacanth, hydroxypropylcellulose, pregelatinized starch, gelatin, hydroxypropylcellulose, hydroxypropylmethylcellulose, methylcellulose, sugar solutions, such as sucrose and sorbitol, and ethylcellulose. Additional agents such as diluents, glidants, coloring agents, and the like, known to a skilled formulator may be combined with the above listed ingredients. Seal coats (*e.g*., Opadry II Blue) may be applied to tablet cores.

As used herein for solid oral dosage forms of the present invention, the term "diluent" with respect to powdered formulations refers to a substance that usually makes up the major portion of the formulation or dosage form. Suitable diluents include sugars such as lactose, sucrose, mannitol, and sorbitol; starches derived from wheat, corn rice, and potato; and celluloses such as microcrystalline cellulose. As exemplified in the formulations 1A-3D, more than one diluent may be used a single formulation. The total amount of diluent in the formulation can range from 60% to 95% by weight of the total formulation, preferably from 80% to 90%.

As used herein for solid oral dosage forms of the present invention, the term "disintegrant" refers to a substance added to the dosage form to help it break apart (disintegrate) and release the medicinal agent(s). Suitable disintegrants include: microcrystalline celluloses and cross-linked celluloses such as sodium croscarmellose; starches; "cold water soluble" modified starches such as sodium carboxymethyl starch; natural and synthetic gums such as locust bean, karaya, guar, tragacanth, and agar; cellulose derivatives such as methylcellulose and sodium carboxymethylcellulose; alginates such as alginic acid and sodium alginate; clays such as bentonites; and effervescent mixtures. Preferred disintegrants comprise croscarmellose sodium, starch, sodium starch glycolate, crospovidone and croscarmelose sodium and microcrystalline cellulose. The amount of disintegrant in the formulation can range from 2% to 12% by weight of the formulation, more preferably from 3.5% to 6% by weight.

As used herein for solid oral dosage forms of the present invention, the term "lubricant" refers to a substance added to the dosage form to enable the tablet after it has been compressed, to release from the mold or die by reducing friction or wear. Suitable lubricants include metallic stearates such as magnesium stearate (vegetable grade), calcium stearate or potassium stearate; stearic acid; high melting point waxes; and water soluble lubricants such as sodium chloride, sodium benzoate, sodium acetate, sodium oleate, polyethylene glycols, and d'I-leucine. Preferred lubricants comprise magnesium stearate, stearic acid and talc. The amount of lubricant in the formulation can range from 0.1 % to 2% by weight of the formulation, preferably 0.5% by weight.

As used herein for solid oral dosage forms of the present invention, the term "glidant" refers to a substance that prevents caking and improves the flow characteristics of granulations, so that flow is smooth and uniform. Suitable glidants include silicon dioxide and talc. The amount of glidant in the formulation can range from 0.1 % to 5% by weight of the total formulation, preferably from 0.5% to 2% by weight.

As used herein for solid oral dosage forms of the present invention, the phrase "coloring agent" refers to a substance that provides coloration to the formulation or the dosage form. Such substances can include food grade dyes and food grade dyes adsorbed onto a suitable adsorbent such as clay or aluminum oxide. The amount of the coloring agent can vary from 0.1 % to 5% by weight of the formulation, preferably from 0.1 % to about 1 %.

The present disclosure encompasses immediate-release tablet formulations of any thrombin receptor antagonist. A variety of compounds have been demonstrated as displaying activity as thrombin receptor antagonists, many being himbacine analogs. As disclosed in U.S. publication no. 04/0152736, a subset of particularly preferred compounds of Formula I is as follows: 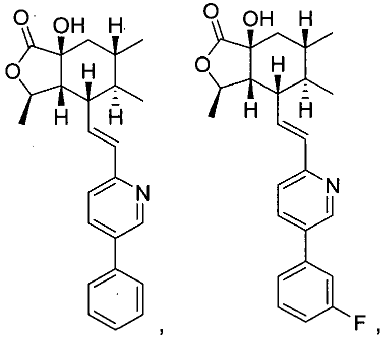 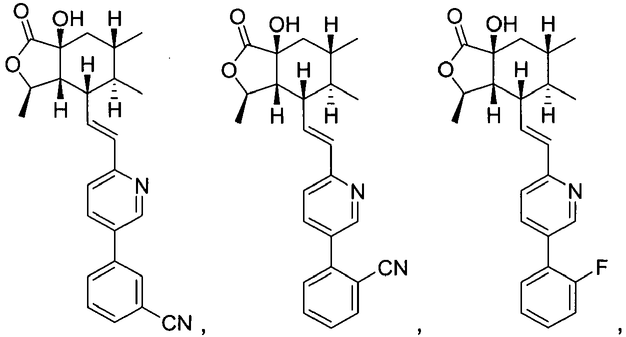 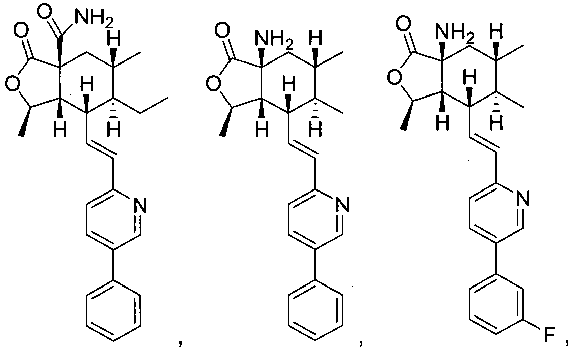 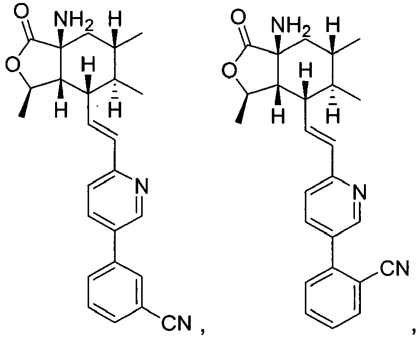 and the pharmaceutically acceptable isomers, salts, solvates and polymorphs thereof. U.S. publication no. 03/0216437 discloses a subset of thrombin receptor antagonists of Formula II which are both particularly active and selective. These compounds are as follows: 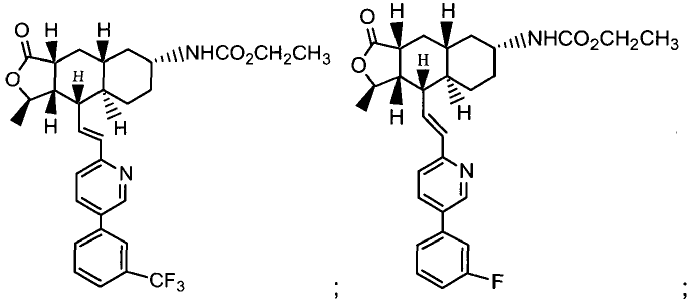 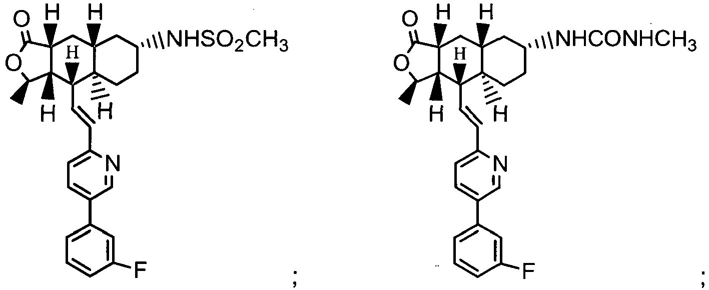 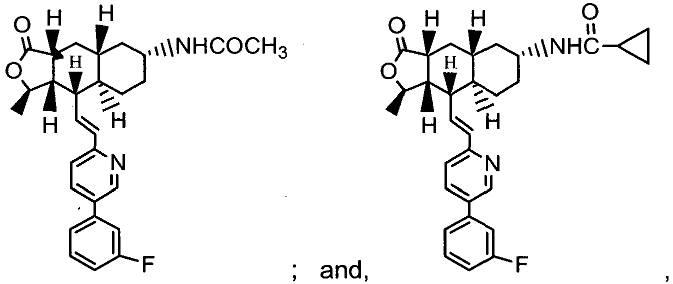 and the pharmaceutically acceptable isomers, salts, solvates and polymorphs thereof.

The following compounds are particularly favored based on their pharmacokinetics and phamacodynamic characteristics: 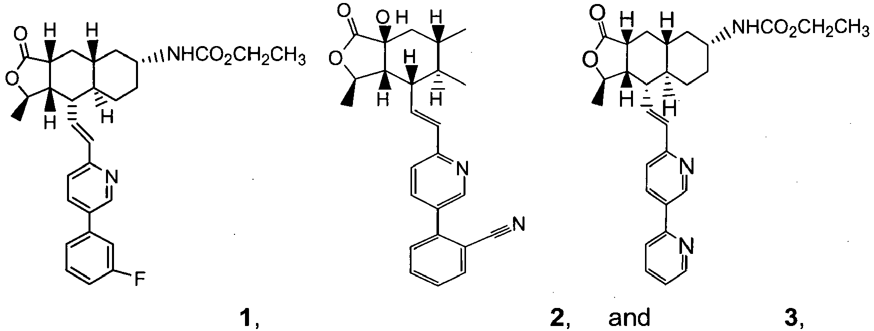 and the pharmaceutically acceptable isomers, salts, solvates and polymorphs thereof. The bisulfate salt of COMPOUND 1 is currently in development as a thrombin receptor antagonist by Schering Corp. Its synthesis is disclosed in U.S. publication no. 03/0216437, published Nov. 20, 2003, which publication also discloses Compound **3.** Compound **2** is disclosed in U.S. Patent no. 6,645,987.

Other compounds for use in the combinations of the present invention are disclosed in any of U.S. Patent Nos. 6,063,847 and 6,326,380, U.S. Patent Publications 03/0203927, 03/0216437, 04/0192753 and 04/0176418. Combinations that include one or more other agents that display activity as thrombin receptor antagonists are also within the scope of the present invention, including E5555 currently in development by Eisai:

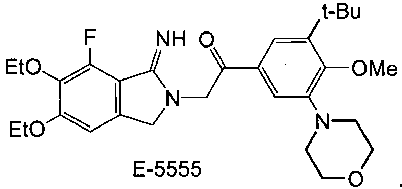

It will be understood that unless otherwise specified, the term "thrombin receptor antagonist:" and any compounds identified as such, including COMPOUND 1, encompasses any chemically stable and pharmaceutically acceptable free base, salt, isomer or solvate form thereof. The term "salt(s)", as employed herein, denotes acidic salts formed with inorganic and/or organic acids. Pharmaceutically acceptable (*i.e*., non-toxic, physiologically acceptable) salts are preferred, although other salts are also useful. Salts of the compound of the above active agents may be formed, for example, by reacting the above active agents with an equivalent amount of acid or base in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates), and the like. Additionally, acids which are generally considered suitable for the formation of pharmaceutically useful salts from basic pharmaceutical compounds are discussed, for example, by S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website). All such acid salts are intended to be pharmaceutically acceptable salts within the scope of the invention and all acid and base salts are considered equivalent to the free forms of the corresponding compounds for purposes of the invention.

All isomers, including diastereomers and rotational isomers are contemplated as being part of this invention. The invention includes (+)- and (-)-isomers in both pure form and in admixture, including racemic mixtures. All stereoisomers (for example, geometric isomers, optical isomers and the like) of the present compounds (including those of the salts and solvates of the compounds), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate," "prodrug" and the like, is intended to equally apply to the salt, solvate and prodrug of enantiomers, stereoisomers, rotamers, tautomers, racemates or prodrugs of the inventive compounds.

The term "solvate" will be understood to encompass hydrates.

Other than as shown in the operating example or as otherwise indicated, all numbers used in the specification and claims expressing quantities of ingredients, reaction conditions, and so forth, are understood as being modified in all instances by the term "about."

## Claims

1. A solid pharmaceutical formulation for oral administration comprising COMPOUND 1 or a pharmaceutically acceptable salt or solvate thereof and at least one disintegrant,
wherein the amount of COMPOUND 1 is less than 7% of the weight of the formulation,
wherein the ratio of disintegrant to COMPOUND 1 or a pharmaceutically acceptable salt or solvate thereof is between 0.6 and 12 on a weight/weight basis,
wherein the weight of COMPOUND 1 or a pharmaceutically acceptable salt or solvate thereof is between 0.5 mg and 10 mg and the total weight of the formulation is between 100 mg and 400 mg, and
wherein COMPOUND 1 is:

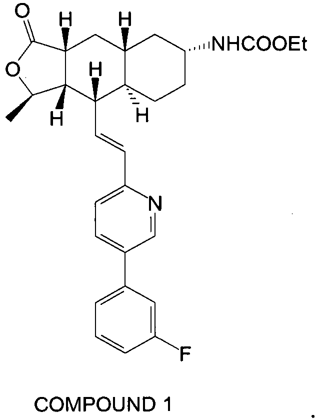

2. The pharmaceutical formulation according to claim 1, wherein said formulation is a tablet.

3. The pharmaceutical formulation according to claim 1, wherein said ratio is between 0.75 and 1.0.

4. The pharmaceutical formulation according to claim 3, wherein said ratio is 0.9.

5. The pharmaceutical formulation according to claim 1, wherein said ratio is 2.4.

6. The pharmaceutical formulation according to claim 1, wherein the weight of COMPOUND 1 or a pharmaceutically acceptable salt or solvate thereof is 2.5 mg and the total weight of the formulation is 100 mg.

7. The pharmaceutical formulation according to claim 1, wherein the COMPOUND 1 is a bisulfate salt.

8. The pharmaceutical formulation according to claim 1, wherein said disintegrant is selected from the group consisting of croscarmellose sodium, starch, sodium starch glycolate, crospovidone and microcrystalline cellulose.

9. The pharmaceutical formulation according to claim 1, wherein said disintegrant is croscarmellose sodium.

10. The pharmaceutical formulation according to claim 1, further comprising at least one diluent, at least one binder and at least one lubricant.

11. The pharmaceutical formulation according to claim 10, wherein said diluent is selected from one or more of the group consisting of lactose monohydrate, microcrystalline cellulose, mannitol, sorbitol, tribasic calcium phosphate, dibasic calcium phosphate, compressible sugar, starch, and calcium sulfate.

12. The pharmaceutical formulation according to claim 10, wherein said diluent is selected from one or more of the group consisting of lactose monohydrate and microcrystalline cellulose.

13. The pharmaceutical formulation according to claim 10, wherein said binder is selected from the group consisting of povidone, acacia, tragacanth, hydroxypropylcellulose, pregelatinized starch, gelatin, hydroxypropylmethylcellulose, methylcellulose, sucrose, sorbitol, and ethylcellulose.

14. The pharmaceutical formulation according to claim 10, wherein said binder is povidone.

15. The pharmaceutical formulation according to claim 10, wherein said lubricant is selected from the group consisting of magnesium stearate, stearic acid and talc.

16. The pharmaceutical formulation according to claim 10, wherein said lubricant is magnesium stearate.

17. The formulation according to claim 1, comprising 2.5 mg of COMPOUND 1 or a pharmaceutically acceptable salt thereof and at least 5 weight percent of a disintegrant, wherein COMPOUND 1 is:

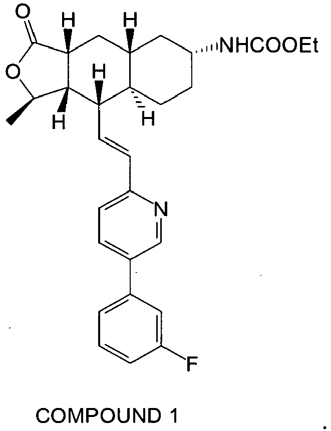

18. The formulation according to claim 17, wherein the total weight of said formulation is 100 mg.

19. The formulation according to claim 17, wherein said formulation is a tablet.

20. The formulation according to claim 1, comprising:
| Ingredient | Amount (mg) |
|---|---|
| COMPOUND 1 Bisulfate | 2.5 |
| Lactose Monohydrate | 68 |
| Microcrystalline Cellulose | 20 |
| Croscarmellose Sodium | 6 |
| Povidone | 3 |
| Magnesium Stearate | 0.5, |
wherein COMPOUND 1 is:

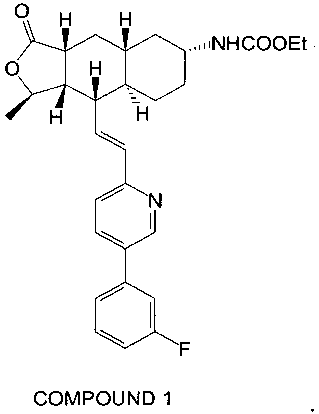

21. A solid pharmaceutical formulation according to any preceding claim for use in a method of treating acute coronary syndrome by orally administering to a patient in need of such treating the pharmaceutical formulation.

22. A solid pharmaceutical formulation according to any of claims 1-10 for use in a method of treating a patient in need of secondary prevention from acute coronary syndrome by orally administering to said patient the pharmaceutical formulation.

23. A solid pharmaceutical formulation according to any of claims 1-20 for use in a method of treating peripheral arterial disease by orally administering to a patient in need of such treating the pharmaceutical formulation.

## Patentansprüche

1. Eine feste pharmazeutische Formulierung zur oralen Verabreichung, umfassend VERBINDUNG 1 oder ein pharmazeutisch annehmbares Salz oder Solvat davon und wenigstens ein Sprengmittel,
wobei die Menge an VERBINDUNG 1 weniger als 7% des Gewichts der Formulierung beträgt,
wobei das Verhältnis von Sprengmittel zu VERBINDUNG 1 oder einem pharmazeutisch annehmbaren Salz oder Solvat davon zwischen 0,6 und 12 auf Gewicht/Gewicht-Basis beträgt,
wobei das Gewicht von VERBINDUNG 1 oder eines pharmazeutisch annehmbaren Salzes oder Solvats davon zwischen 0,5 mg und 10 mg beträgt und das Gesamtgewicht der Formulierung zwischen 100 mg und 400 mg beträgt, und
wobei VERBINDUNG 1 ist:

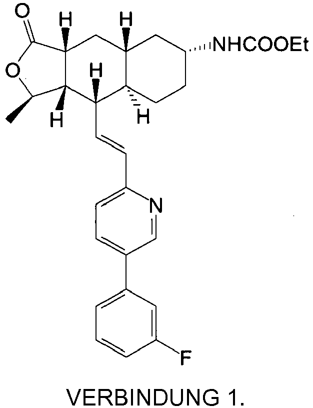

2. Die pharmazeutische Formulierung gemäß Anspruch 1, wobei die Formulierung eine Tablette ist.

3. Die pharmazeutische Formulierung gemäß Anspruch 1, wobei das Verhältnis zwischen 0,75 und 1,0 beträgt.

4. Die pharmazeutische Formulierung gemäß Anspruch 3, wobei das Verhältnis 0,9 beträgt.

5. Die pharmazeutische Formulierung gemäß Anspruch 1, wobei das Verhältnis 2,4 beträgt.

6. Die pharmazeutische Formulierung gemäß Anspruch 1, wobei das Gewicht der VERBINDUNG 1 oder eines pharmazeutisch annehmbaren Salzes oder Solvats davon 2,5 mg beträgt und das Gesamtgewicht der Formulierung 100 mg beträgt.

7. Die pharmazeutische Formulierung gemäß Anspruch 1, wobei die VERBINDUNG 1 ein Bisulfatsalz ist.

8. Die pharmazeutische Formulierung gemäß Anspruch 1, wobei das Sprengmittel ausgewählt ist aus der Gruppe bestehend aus Croscarmellose-Natrium, Stärke, Natriumstärkeglykolat, Crospovidon und mikrokristalliner Cellulose.

9. Die pharmazeutische Formulierung gemäß Anspruch 1, wobei das Sprengmittel Croscarmellose-Natrium ist.

10. Die pharmazeutische Formulierung gemäß Anspruch 1, ferner umfassend wenigstens ein Verdünnungsmittel, wenigstens ein Bindemittel und wenigstens ein Gleitmittel.

11. Die pharmazeutische Formulierung gemäß Anspruch 10, wobei das Verdünnungsmittel ausgewählt ist aus einem oder mehreren aus der Gruppe bestehend aus Lactose-Monohydrat, mikrokristalliner Cellulose, Mannit, Sorbit, Tricalciumphosphat, Calciumhydrogenphosphat, verpressbarem Zucker, Stärke und Calciumsulfat.

12. Die pharmazeutische Formulierung gemäß Anspruch 10, wobei das Verdünnungsmittel ausgewählt ist aus einem oder mehreren aus der Gruppe bestehend aus Lactose-Monohydrat und mikrokristalliner Cellulose.

13. Die pharmazeutische Formulierung gemäß Anspruch 10, wobei das Bindemittel ausgewählt ist aus der Gruppe bestehend aus Povidon, Gummi arabicum, Tragant, Hydroxypropylcellulose, Quellstärke, Gelatine, Hydroxypropylmethylcellulose, Methylcellulose, Saccharose, Sorbit und Ethylcellulose.

14. Die pharmazeutische Formulierung gemäß Anspruch 10, wobei das Bindemittel Povidon ist.

15. Die pharmazeutische Formulierung gemäß Anspruch 10, wobei das Gleitmittel ausgewählt ist aus der Gruppe bestehend aus Magnesiumstearat, Stearinsäure und Talk.

16. Die pharmazeutische Formulierung gemäß Anspruch 10, wobei das Gleitmittel Magnesiumstearat ist.

17. Die Formulierung gemäß Anspruch 1, umfassend 2,5 mg VERBINDUNG 1 oder eines pharmazeutisch annehmbaren Salzes davon und wenigstens 5 Gew.-% eines Sprengmittels, wobei VERBINDUNG 1 ist:

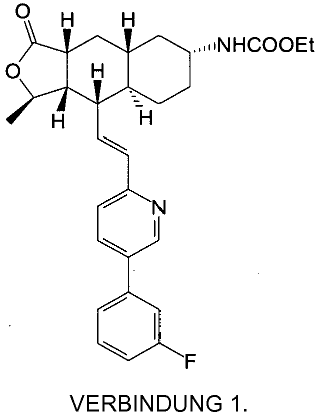

18. Die Formulierung gemäß Anspruch 17, wobei das Gesamtgewicht der Formulierung 100 mg beträgt.

19. Die Formulierung gemäß Anspruch 17, wobei die Formulierung eine Tablette ist.

20. Die Formulierung gemäß Anspruch 1, umfassend:
| Bestandteil | Menge (mg) |
|---|---|
| VERBINDUNG 1 Bisulfat | 2,5 |
| Lactose-Monohydrat | 68 |
| Mikrokristalline Cellulose | 20 |
| Croscarmellose-Natrium | 6 |
| Povidon | 3 |
| Magnesiumstearat | 0,5, |
wobei VERBINDUNG 1 ist:

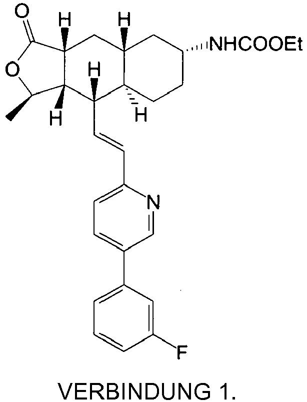

21. Eine feste pharmazeutische Formulierung gemäß einem vorhergehenden Anspruch zur Verwendung bei einem Verfahren zur Behandlung von akutem Koronarsyndrom durch orale Verabreichung der pharmazeutischen Formulierung an einen Patienten, der eine solche Behandlung benötigt.

22. Eine feste pharmazeutische Formulierung gemäß einem der Ansprüche 1-10 zur Verwendung bei einem Verfahren zur Behandlung eines Patienten, der eine sekundäre Vorbeugung gegen akutes Koronarsyndrom benötigt, durch orale Verabreichung der pharmazeutischen Formulierung an den Patienten.

23. Eine feste pharmazeutische Formulierung gemäß einem der Ansprüche 1-20 zur Verwendung bei einem Verfahren zur Behandlung von peripherer arterieller Verschlusskrankheit durch orale Verabreichung der pharmazeutischen Formulierung an einen Patienten, der eine solche Behandlung benötigt.

## Revendications

1. Formulation pharmaceutique solide pour une administration orale, comprenant le COMPOSE 1 ou un sel ou un solvate pharmaceutiquement acceptable de celui-ci et au moins un désintégrant,
dans laquelle la quantité du COMPOSE 1 est de moins de 7% du poids de la formulation,
dans laquelle le rapport du désintégrant sur le COMPOSE 1 ou un sel ou un solvate pharmaceutiquement acceptable de celui-ci est entre 0,6 et 12 sur une base poids/poids,
dans laquelle le poids du COMPOSE 1 ou un sel ou un solvate pharmaceutiquement acceptable de celui-ci est entre 0,5 et 10 mg et le poids total de la formulation est entre 100 mg et 400 mg, et
dans laquelle le COMPOSE 1 est:

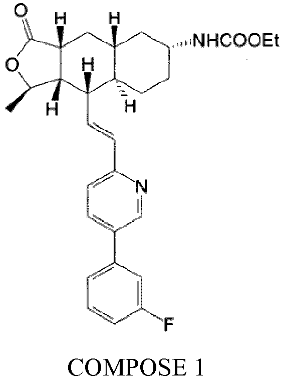

2. Formulation pharmaceutique selon la revendication 1, dans laquelle ladite formulation est un comprimé.

3. Formulation pharmaceutique selon la revendication 1, dans laquelle ledit rapport est entre 0,75 et 1,0.

4. Formulation pharmaceutique selon la revendication 3, dans laquelle ledit rapport est de 0,9.

5. Formulation pharmaceutique selon la revendication 1, dans laquelle ledit rapport est de 2,4.

6. Formulation pharmaceutique selon la revendication 1, dans laquelle le poids du COMPOSE 1 ou un sel ou un solvate pharmaceutiquement acceptable de celui-ci est de 2,5 mg et le poids total de la formulation est de 100 mg.

7. Formulation pharmaceutique selon la revendication 1, dans laquelle le COMPOSE 1 est un sel de bisulfate.

8. Formulation pharmaceutique selon la revendication 1, dans laquelle ledit désintégrant est choisi dans le groupe constitué de: croscarmellose sodique, amidon, glycolate de sodium et d'amidon, crospovidone et cellulose microcristalline.

9. Formulation pharmaceutique selon la revendication 1, dans laquelle ledit désintégrant est le croscarmellose sodique.

10. Formulation pharmaceutique selon la revendication 1, comprenant en outre au moins un diluant, au moins un liant et au moins un lubrifiant.

11. Formulation pharmaceutique selon la revendication 10, dans laquelle ledit diluant est choisi parmi un ou plusieurs du groupe constitué de: monohydrate de lactose, cellulose microcristalline, mannitol, sorbitol, phosphate de calcium tribasique, phosphate de calcium dibasique, sucre compressible, amidon et sulfate de calcium.

12. Formulation pharmaceutique selon la revendication 10, dans laquelle ledit diluant est sélectionné parmi un ou plusieurs du groupe constitué de monohydrate de lactose et de cellulose microcristalline.

13. Formulation pharmaceutique selon la revendication 10, dans laquelle ledit liant est choisi dans le groupe constitué de: povidone, acacia, tragacanthe, hydroxypropylcellulose, amidon prégélatinisé, gélatine, hydroxypropylméthylcellulose, méthylcellulose, saccharose, sorbitol et éthylcellulose.

14. Formulation pharmaceutique selon la revendication 10, dans laquelle ledit liant est la povidone.

15. Formulation pharmaceutique selon la revendication 10, dans laquelle ledit lubrifiant est choisi dans le groupe constitué de: stéarate de magnésium, acide stéarique et talc.

16. Formulation pharmaceutique selon la revendication 10, dans laquelle ledit lubrifiant est le stéarate de magnésium.

17. Formulation selon la revendication 1, comprenant 2,5 mg du COMPOSE 1 ou un sel pharmaceutiquememt acceptable de celui-ci et au moins 5 pourcent en poids d'un désintégrant, dans laquelle le COMPOSE 1 est :

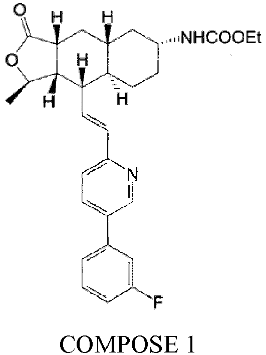

18. Formulation selon la revendication 17, dans laquelle le poids total de ladite formulation est de 100 mg.

19. Formulation pharmaceutique selon la revendication 17, dans laquelle ladite formulation est un comprimé.

20. Formulation pharmaceutique selon la revendication 1, comprenant:
| Ingrédient | Quantité (mg) |
|---|---|
| Bisulfate du COMPOSE 1 | 2,5 |
| Monohydrate de lactose | 68 |
| Cellulose microcristalline | 20 |
| Croscarmellose sodique | 6 |
| Povidone | 3 |
| Stéarate de magnésium | 0,5 |
dans laquelle le COMPOSE 1 est:

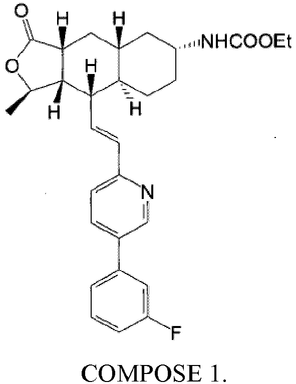

21. Formulation pharmaceutique solide selon l'une quelconque des revendications précédentes pour une utilisation dans une méthode de traitement d'un syndrome coronarien aigu en administrant oralement à un patient ayant besoin d'un tel traitement la formulation pharmaceutique.

22. Formulation pharmaceutique solide selon l'une quelconque des revendications 1-10 pour une utilisation dans une méthode de traitement d'un patient ayant besoin d'une prévention secondaire d'un syndrome coronarien aigu en administrant oralement audit patient la formulation pharmaceutique.

23. Formulation pharmaceutique solide selon l'une quelconque des revendications 1-20 pour une utilisation dans une méthode de traitement d'une maladie artérielle périphérique en administrant oralement à un patient ayant besoin d'un tel traitement la formulation pharmaceutique.
